# FASCICULE DE BREVET EUROPEEN

(11) **EP 0 626 182 B1**
(45) Date de publication et mention de la délivrance du brevet: **04.08.1999**
(21) Numéro de dépôt: 94401055.2
(22) Date de dépôt: 11.05.1994
(51) Int. Cl.: A61N 1/368, A61N 1/39

(54) **Dispositif d'analyse de l'activité cardiaque pour déterminer si une tachyarythmie est susceptible d'être interrompue par stimulation**
Vorrichtung zur Herztätigkeit-Analyse zur Bestimmung, ob eine Tachyarrythmie durch Stimulation unterbrochen werden kann
Device for analysis of the cardiac activity for determining whether or not a tachyarrythmia may be interrupted by stimulation

(30) Priorité: 28.05.1993 FR 9306406
(43) Date de publication de la demande: 30.11.1994
(73) Titulaire: ELA MEDICAL (Société anonyme), F-92541 Montrouge Cédex (FR)
(72) Inventeur: Jacobson, Peter, F-67500 Haguenau (FR); Kroiss, Daniel, F-67590 Schweighouse-Moder (FR); Henry, Christine, F-75014 Paris (FR)
(74) Mandataire: Laget, Jean-Loup

(56) Documents cités:
- EP-A- 0 540 141
- WO-A-93/02746
- US-A- 4 860 749
- US-A- 5 107 850

## Description

L'invention concerne un dispositif d'analyse de l'activité cardiaque pour déterminer si une tachyarythmie est susceptible d'être interrompue par stimulation.

Elle se rapporte aux dispositifs médicaux qui surveillent l'état cardiaque d'un patient en détectant les signaux auriculaires et ventriculaires, et qui analysent ces signaux pour déterminer si une tachyarythmie est présente, et dans ce cas, pour déterminer si cette tachyarythmie est d'un type susceptible d'être interrompu par une stimulation antitachycardique.

La stimulation antitachycardique (ATP) recouvre tout mode de stimulation destiné à interrompre la tachyarythmie, y compris la stimulation ventriculaire et/ou auriculaire. La tachyarythmie correspond à un rythme cardiaque rapide anormal, et recouvre la fibrillation ventriculaire (VF), la tachycardie ventriculaire (VT), la tachycardie sinusale (ST) et la tachycardie supra-ventriculaire (SVT). La tachycardie supra-ventriculaire recouvre la tachycardie auriculaire, le flutter auriculaire, et la fibrillation auriculaire.

Un rythme normal sans tachyarythmie est appelé rythme sinusal (SR).

Les dispositifs médicaux en question mesurent directement ou indirectement l'intervalle entre signaux ventriculaires (RR), entre signaux auriculaires (PP), entre un signal auriculaire et un signal ventriculaire (PR), ou entre un signal ventriculaire et un signal auriculaire (RP). Ils comptent, ensemble ou séparément, le nombre des signaux auriculaires (Na) ou ventriculaires (Nv) pendant un nombre prédéterminé de cycles cardiaques.

Plus particulièrement, l'invention se rapporte à des dispositifs médicaux qui lorsqu'ils sont appliqués dans le ventricule, identifient d'abord la fibrillation ventriculaire (VF), lorsque la fréquence ventriculaire est supérieure à une première fréquence prédéterminée, et le rythme sinusal (SR), lorsque la fréquence ventriculaire est inférieure à une deuxième fréquence prédéterminée, et ensuite s'efforcent de classer les autres rythmes dont les fréquences sont situées entre ces deux fréquences prédéterminées.

En conséquence, l'invention s'applique aux dispositifs qui s'efforcent de distinguer la tachycardie ventriculaire (VT) de la tachycardie sinusale (ST) ou de la tachycardie supra-ventriculaire (SVT), dans une gamme intermédiaire de fréquences.

L'invention vise à déterminer si une tachyarythmie est susceptible d'être interrompue par stimulation dans l'oreillette ou dans le ventricule.

Elle s'applique aux dispositifs qui délivrent une thérapie après avoir analysé le rythme cardiaque, et à ceux qui ne le font pas. Elle s'applique aux dispositifs implantables ou externes. Elle s'applique à l'analyse du rythme cardiaque avant, pendant et après la thérapie.

La notion de thérapie recouvre la stimulation antitachycardie (ATP), la cardioversion et la défibrillation.

Selon Arzbaecher et al., Pace, Vol. 7, pp 541-547 (1984), la détection des signaux cardiaques dans une seule chambre pour déterminer l'instant d'application de la stimulation antitachycardique (ATP) dans cette chambre, peut conduire à une stimulation inappropriée et à une accélération subséquente du rythme cardiaque, ou même au déclenchement d'une tachycardie. Ce document propose la détection des signaux auriculaires et ventriculaires ainsi que la division en trois zones des tachyarythmies de fréquence intermédiaire :
- lorsque Nv >> Na, il y a VT
- lorsque Nv << Na, il y a ST ou SVT
- lorsque Nv ≈ Na, l'un des deux critères de la VT est appliqué :
   . Accélération du rythme ventriculaire excédant une limite prédéterminée,
   . Changement du rythme ventriculaire à la suite d'un stimulus auriculaire prématuré, n'excédant pas une limite prédéterminée.

Selon Schuger et al., Pace, Vol. 11, pp 1456-1464 (1988), la tachycardie ventriculaire (VT) est caractérisée par un critère : l'intervalle RR est stable, et l'intervalle PR est instable. La moyenne des mesures de RR et PR sur plusieurs cycles permet d'éviter de satisfaire au critère de tachycardie ventriculaire (VT) en cas d'extrasystole ventriculaire.

Le document US-A-4 860 749, Lehmann, prévoit la division en trois zones des rythmes ventriculaires à fréquence intermédiaire (qui correspondent respectivement aux trois zones prévues par Arzbaecher) :
- lorsque RR < PP, il y a VT ;
- lorsque RR > PP, le critère de VT est appliqué : RR stable et PR instable ;
- lorsque RR = PP, le critère de VT est appliqué : PR plus long que PR en rythme sinusal.

La détection des signaux auriculaires et ventriculaires peut améliorer la spécificité de la détection, dans une gamme de fréquences intermédiaires, des tachyarythmies susceptibles d'être interrompues par stimulation. Le critère de stabilité pour la détermination des tachyarythmies ventriculaires susceptibles d'être interrompues par stimulation, consiste à déterminer s'il y a stabilité de l'intervalle RR et instabilité de l'intervalle PR.

Ce procédé part de la supposition que dans une tachycardie ventriculaire (VT) susceptible d'être interrompue par stimulation, la plupart des signaux ventriculaires sont provoqués par un signal ventriculaire antérieur, conduit par un chemin circulaire, avec un temps de conduction stable.

Inversement, lorsque la plupart des signaux ventriculaires sont provoqués par des signaux auriculaires, conduits par un chemin auriculo-ventriculaire, avec un temps de conduction stable, alors il n'y a pas de tachycardie ventriculaire (VT) susceptible d'être interrompue par stimulation.

Selon l'état de la technique, ce critère s'applique lorsqu'il n'y a pas d'association auriculo-ventriculaire en 1 : 1. Lorsqu'il y a association en 1 : 1, le critère ne peut pas distinguer une tachyarythmie auriculaire conduite au ventricule, d'une tachyarythmie ventriculaire conduite à l'oreillette.

L'état de la technique présente des inconvénients, en particulier en ce qui concerne le calcul et l'application du critère de stabilité.

Le brevet US-A-4 860 749 ne définit pas le signal auriculaire à prendre en compte pour le calcul de l'intervalle PR. Lorsque Na > Nv, plus d'un signal auriculaire peut être détecté par cycle ventriculaire. Si plus d'un intervalle PR par cycle ventriculaire est pris en compte dans un calcul de stabilité par moyenne, l'intervalle PR apparaît comme instable, même s'il y a une conduction auriculo-ventriculaire avec un bloc fixe et un intervalle PR très stable.

L'état de la technique applique le critère de stabilité seulement lorsque Na > Nv. Si l'on considère le démarrage d'une tachycardie sinusale (ST) avec Na = Nv, l'intervalle RR diminue mais l'intervalle PR reste constant. Le critère de stabilité pourrait être appliqué pour conclure, correctement, qu'il n'y a pas de tachycardie ventriculaire (VT).

Le brevet US-A-4 860 749 ne donne pas d'indication sur le calcul de l'instabilité de PR ou de la stabilité de RR, alors que la publication Schuger et al. suggère d'utiliser des moyennes sur plusieurs cycles cardiaques. Cette technique est affectée par la détection d'une extrasystole, d'un train d'interférences électriques, ou par la non-détection d'un événement cardiaque.

Le critère de stabilité devrait pouvoir être appliqué à la détection des tachyarythmies susceptibles d'être interrompues par stimulation, aussi bien dans l'oreillette que dans le ventricule.

La présente invention a pour but d'améliorer la spécificité de la détection dans une première chambre des tachyarythmies susceptibles d'être interrompues par stimulation, en proposant un critère fondé sur la stabilité dans cette première chambre et l'instabilité de la conduction en provenance de la seconde chambre.

A cet effet, le dispositif de l'invention :
- prendre en compte tous les signaux détectés dans la seconde chambre, qui pourraient avoir donné lieu à un signal détecté dans la première chambre, et pas seulement le signal dans la seconde chambre précédant immédiatement le signal dans la première chambre ;
- appliquer le critère à tous les rythmes dans la première chambre, y compris les rythmes avec association en 1 : 1, pour définir que les rythmes sans stabilité dans cette chambre ne sont pas susceptibles d'être interrompus par stimulation ,
- calculer la stabilité de manière telle que quelques signaux prématurés, l'incidence du bruit électrique, ou des signaux non détectés dans l'une ou l'autre chambre, n'affectent pas le calcul de façon importante ;
- utilise un procédé applicable à chaque chambre.

Selon l'invention, le dispositif maintient un histogramme des intervalles récents dans la première chambre, et un histogramme des intervalles récents de conduction en provenance de la seconde chambre, relatifs aux mêmes événements, ces intervalles étant triés dans des cases en fonction de leur longueur.

Il maintient un total d'autocorrélation et un total d'intercorrélation, correspondant au nombre total de comptages dans l'histogramme respectif.

Il utilise les histogrammes pour évaluer les critères suivants :
- Pic d'autocorrélation : le nombre maximal d'intervalles récents dans la première chambre qui satisfont à un critère de stabilité prédéterminé ;
- Pic d'intercorrélation : le nombre maximal d'intervalles de conduction en provenance de la seconde chambre, qui satisfont à un critère de stabilité de conduction prédéterminé.

Il définit qu'il y a stabilité dans la première chambre lorsque le pic d'autocorrélation, divisé par le total d'autocorrélation, excède un rapport prédéterminé.

Il définit qu'il y a stabilité de conduction lorsque le pic d'intercorrélation, divisé soit par le pic d'autocorrélation (dans le première variante de réalisation de l'invention) soit par le total d'intercorrélation (dans la seconde variante) excède un rapport prédéterminé.

La première variante de réalisation de l'invention compare la stabilité de la conduction entre les deux chambres à celle des intervalles dans la première chambre.

La seconde variante exprime la stabilité de la conduction entre les deux chambres en fonction de la totalité des conductions présumées.

Lorsqu'il n'y a pas de stabilité dans la première chambre, cela signifie que la tachyarythmie n'est probablement pas susceptible d'être interrompue par stimulation dans la première chambre, et le procédé définit qu'elle n'est pas susceptible d'être interrompue dans cette chambre.

Lorsqu'il y a stabilité dans la première chambre, mais pas de stabilité de conduction, cela signifie que la tachyarythmie a probablement son origine dans la première chambre, et le procédé définit qu'elle est susceptible d'être interrompue par stimulation dans cette chambre.

Lorsqu'il y a stabilité dans la première chambre et stabilité de conduction, le procédé doit déterminer si la conduction en provenance de la seconde chambre est en 1 : 1 ou en n : 1. Lorsque le pic d'intercorrélation, divisé par le total d'intercorrélation, excède un rapport prédéterminé, le procédé définit que l'association est en 1 : 1.

En variante, lorsque le nombre d'événements comptés dans la seconde chambre divisé par le nombre d'événements comptés dans la première chambre est supérieur à un rapport prédéterminé, de préférence égal à 1,33, le procédé définit que l'association est en n : 1.

Dans le cas d'une association en 1 : 1; le procédé utilise un autre critère, par exemple la présence d'une accélération prenant naissance avec une dissociation, pour déterminer si la tachyarythmie est susceptible d'être interrompue par stimulation.

Dans le cas d'une association en n : 1, le procédé conclut que la tachyarythmie n'est pas susceptible d'être interrompue par stimulation dans la première chambre, parce qu'elle a son origine dans la seconde.

La présente invention a pour objet un dispositif d'analyse de l'activité cardiaque par détection des signaux auriculaires et ventriculaires, correspondant à des événements cardiaques, pour déterminer si une tachyarythmie est susceptible d'être interrompue par stimulation, caractérisé en ce qu'il comporte :
- la détermination du nombre maximal, appelé pic d'autocorrélation, des intervalles récents entre les signaux cardiaques détectés dans une première chambre cardiaque, satisfaisant à des critères prédéterminés de stabilité, et
- la détermination du nombre maximal, appelé pic d'intercorrélation, des signaux cardiaques récents détectés dans ladite première chambre cardiaque et correspondant aux mêmes événements que précédemment, qui sont précédés par des signaux cardiaques détectés dans la seconde chambre, dans des limites prédéterminées de temps de conduction.

Selon d'autres caractéristiques avantageux de l'invention :
- la détermination du pic d'autocorrélation comprend les étapes suivantes :
   . maintien d'un histogramme d'autocorrélation, c'est-à-dire d'un histogramme des intervalles récents dans ladite première chambre ;
   . maintien d'un total d'autocorrélation, c'est-à-dire de la somme du nombre total des comptages dans l'histogramme d'autocorrélation, et
   . balayage de l'histogramme d'autocorrélation avec une fenêtre prédéterminée de stabilité pour déterminer le nombre maximal de comptages dans la fenêtre pour toutes les positions possibles de la fenêtre dans l'histogramme.
- la détermination du pic d'intercorrélation comprend les étapes suivantes :
   . maintien d'un histogramme d'intercorrélation, c'est-à-dire d'un histogramme des intervalles récents de conduction de ladite seconde chambre vers ladite première chambre, correspondant aux mêmes événements que ceux de l'histogramme d'autocorrélation,
   . maintien d'un total d'intercorrélation, c'est-à-dire de la somme du nombre total de comptages dans l'histogramme d'intercorrélation, et
   . balayage de l'histogramme d'intercorrélation avec une fenêtre prédéterminée de temps de conduction pour déterminer le nombre maximal de comptages dans la fenêtre pour toutes les positions possibles de la fenêtre dans l'histogramme.
- l'étape de maintien dudit histogramme des intervalles récents comprend les étapes suivantes :
   . soustraction dans l'histogramme de tout comptage correspondant à un intervalle survenu antérieurement à un nombre prédéterminé de cycles, un cycle correspondant à un signal détecté dans ladite première chambre, et
   . addition à l'histogramme d'un comptage correspondant à chaque intervalle survenu pendant le cycle le plus récent.
- le nombre prédéterminé de cycles est programmable ;
- le nombre prédéterminé de cycles est d'environ 16 ;
- dans l'histogramme d'autocorrélation, les intervalles récents dans la première chambre sont triés dans des cases en fonction de leur longueur ;
- dans l'histogramme d'intercorrélation, les intervalles récents de conduction de la seconde chambre vers la première chambre sont triés dans des cases en fonction de leur longueur ;
- ladite fenêtre consiste en un nombre programmable de cases contiguës ;
- ladite fenêtre consiste en un nombre de cases contiguës correspondant à une variation d'intervalle d'environ 64 ms ;
- la largeur d'une case dans un histogramme est d'environ 16 ms ;
- ladite analyse n'est pas effectuée pour les intervalles dans ladite première chambre qui sont plus longs qu'une limite prédéterminée ;
- ladite limite est programmable ;
- ladite limite est d'environ 600 ms ;
- les histogrammes sont remis à zéro lorsqu'aucun événement n'y a été inscrit pendant un nombre prédéterminé de cycles dans ladite première chambre ;
- ledit nombre prédéterminé de cycles est programmable ;
- ledit nombre prédéterminé de cycles est d'environ 16 ;
- l'histogramme d'autocorrélation comprend tous les intervalles entre environ 125 ms et environ 600 ms ;
- l'histogramme d'intercorrélation comprend tous les intervalles de conduction entre environ 16 et environ 500 ms ;
- le procédé comprend une étape de triage des rythmes cardiaques en fonction du pic d'autocorrélation, comprenant elle-même les étapes de :
   . définir qu'il y a stabilité dans la première chambre lorsque le pic d'autocorrélation, divisé par le total d'autocorrélation excède un rapport prédéterminé de stabilité, et
   . définir que la tachyarythmie n'est pas susceptible d'être interrompue par stimulation dans la première chambre lorsqu'il n'y a pas de stabilité dans la première chambre.
- le procédé comprend une étape de triage des rythmes cardiaques en fonction du pic d'autocorrélation et du pic d'intercorrélation, comprenant elle-même les étapes de :
   . définir qu'il y a instabilité de conduction lorsque le pic d'intercorrélation, divisé par le pic d'autocorrélation, n'excède pas un rapport prédéterminé de conduction, et
   . définir que la tachyarythmie est susceptible d'être interrompue par stimulation dans la première chambre lorsqu'il y a stabilité dans la première chambre et instabilité de conduction.
- le procédé comprend une étape de triage des rythmes cardiaques en fonction des valeurs relatives du pic d'intercorrélation et du total d'autocorrélation, comprenant elle-même les étapes de :
   . définir qu'il y a instabilité de conduction lorsque le pic d'intercorrélation, divisé par le total d'autocorrélation, n'excède pas un rapport prédéterminé de conduction, et
   . définir que la tachyarythmie est susceptible d'être interrompue par stimulation dans la première chambre lorsqu'il y a stabilité dans la première chambre et instabilité de conduction.
- le procédé comprend l'étape de trier encore les rythmes cardiaques avec une stabilité définie dans la première chambre et une stabilité de conduction définie, en définissant en outre qu'il y a une conduction en 1 : 1 lorsque le pic d'intercorrélation, divisé par le total d'intercorrélation excède un rapport prédéterminé ;
- le procédé comprend l'étape de trier encore les rythmes cardiaques avec une stabilité définie dans la première chambre, en définissant en outre qu'il y a une conduction en n : 1 lorsque le total des événements dans la deuxième chambre, divisé par le total d'autocorrélation excède un rapport prédéterminé ;
- lesdits rapports prédéterminés sont programmables ;
- le procédé comprend l'étape de triage des rythmes cardiaques seulement lorsque la longueur des intervalles dans ladite première chambre tombe entre une limite inférieure prédéterminée et une limite supérieure prédéterminée ;
- lesdites limites des intervalles sont programmables ;
- la limite inférieure prédéterminée est d'environ 375 ms et la limite supérieure prédéterminée est d'environ 600 ms ;
- le procédé comprend l'étape d'attendre pour trier les rythmes jusqu'à ce que les histogrammes aient été mis à jour pour un nombre minimal prédéterminé de cycles ;
- ledit nombre prédéterminé de cycles est programmable ;
- ledit nombre prédéterminé de cycles est d'environ 8 ;
- le procédé comprend l'étape de trier encore les rythmes cardiaques avec stabilité définie dans la première chambre et stabilité de conduction définie, comprenant elle-même les étapes de :
   . identifier l'occurrence d'une accélération de la fréquence dans la première chambre, excédant une limite d'accélération prédéterminée ;
   . identifier l'occurrence d'une dissociation de conduction entre les deux chambres lors de la détection de l'accélération, et
   . définir que la tachyarythmie est susceptible d'être interrompue par stimulation dans la première chambre lorsqu'il y a accélération et dissociation;
- le procédé comprend l'étape de définir que la tachyarythmie est susceptible d'être interrompue par stimulation seulement lorsqu'elle est définie en 1 : 1.
- le procédé comprend l'étape d'application d'une stimulation antitachycardique à une tachyarythmie définie comme susceptible d'être interrompue par stimulation.

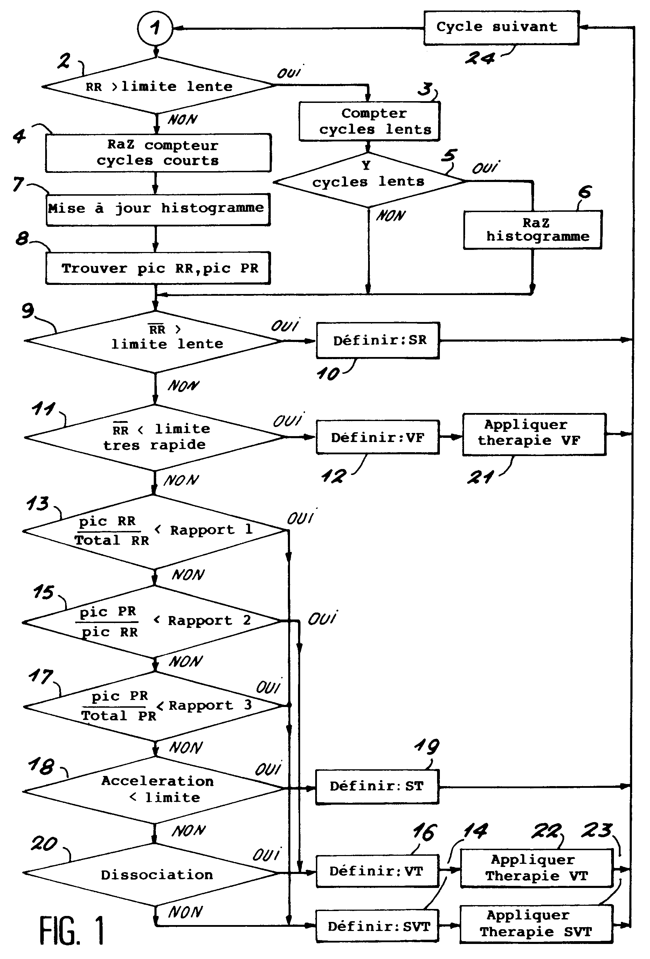

La figure 1 représente le schéma logique de décision du procédé de commande selon l'invention.

L'invention est maintenant décrite dans un exemple de détermination des tachycardies susceptibles d'être interrompues par stimulation dans le ventricule. Elle s'applique également à la détermination des tachycardies susceptibles d'être interrompues par stimulation dans l'oreillette.

Sur la figure 1, le chemin de décision du procédé de commande utilisée dans le dispositif selon l'invention commence au point 1 à la suite de la détection d'une onde R.

En 2, l'intervalle RR est comparé à une limite prédéterminée "lente" qui peut être programmable et qui est de préférence de 600 ms environ. Si la longueur de l'intervalle RR excède la limite "lente" le rythme cardiaque est probablement un rythme sinusal RS. Dans ce cas, un compteur de cycles lents, est incrémenté en 3, dans le cas contraire, le compteur est remis à zéro en 4.

Lorsqu'un nombre prédéterminé Y de cycles lents consécutifs est atteint en 5, chaque case des histogrammes est remise à zéro en 6, étant donné que l'information stockée n'est plus utile. Le nombre Y peut être programmable, et il est de préférence égal à 16.

De cette manière, les étapes de mise à jour et d'analyse des histogrammes, qui consomment du temps et de la puissance, sont accomplies seulement lorsqu'une tachycardie risque d'être présente.

Un histogramme des intervalles RR, et un histogramme des intervalles PR sont mis à jour en 7 :
- dans l'histogramme des intervalles RR, la case correspondant à l'intervalle RR qui vient juste de s'écouler est incrémentée, et chaque case qui avait été incrémentée Z cycles plus tôt, est décrémentée. Le nombre Z peut être programmable et il est de préférence égal à 16 ;
- dans l'histogramme des intervalles PR, une case correspondant à chacun des intervalles PR dans le dernier cycle RR est incrémentée : une case pour chaque onde P détectée. Chaque case qui avait été incrémentée Z cycles plus tôt, est décrémentée.

De cette manière, les histogrammes stockent l'information pour les Z cycles les plus récents pendant lesquels le rythme ventriculaire était plus rapide que la limite lente.

Le pic d'autocorrélation (pic RR) et le pic d'intercorrélation (pic PR) sont définis en 8. Le pic est trouvé par balayage de toutes les cases de l'histogramme avec une fenêtre dont la largeur peut être programmable et est de préférence égale à 64 ms.

Chaque fois que la fenêtre se déplace d'une case, le total des comptages de toutes les cases de la fenêtre est calculé. Le total le plus élevé de toutes les positions de la fenêtre est conservé. De cette façon, le pic est le nombre total maximal des comptages dans chaque jeu de cases contiguës se trouvant dans la fenêtre.

Un intervalle moyen RR est comparé en 9 à une limite "lente" prédéterminée, qui peut être programmable et qui est de préférence égale à 600 ms. Si l'intervalle moyen RR est plus grand que la limite "lente", alors le procédé selon l'invention définit en 10 que le rythme est sinusal (SR).

L'intervalle moyen RR est comparé en 11 à une limite "très rapide", qui peut être programmable, et qui est de préférence égale à 375 ms. Si l'intervalle moyen RR est plus court que la limite "très rapide", alors le procédé selon l'invention définit en 12 qu'il y a fibrillation ventriculaire (VF).

Un intervalle moyen RR est utilisé en 9 et 11 pour éviter que les extrasystoles isolées, les ondes non détectées, et le bruit électrique, n'affectent globalement la décision. A cet effet, une moyenne glissante, un filtre passe-bas, un détecteur en X/Y, ou un autre filtre conventionnel peut être utilisé.

Le pic RR est divisé en 13 par le total de RR. Si le rapport est inférieur à un rapport prédéterminé, qui peut être programmable et qui est de préférence égal à 75%, alors il est peu probable qu'il y ait une conduction selon une voie déterminée à partir d'un événement ventriculaire détecté, pour provoquer l'événement ventriculaire suivant. L'algorithme définit en 14 qu'il y a tachycardie supraventriculaire (SVT).

Le pic PR est divisé en 15 par le pic RR. Si le rapport est inférieur à un rapport prédéterminé, qui peut être programmable et qui est de préférence égal à 75%, alors il est moins probable qu'il y ait conduction de l'oreillette au ventricule que du ventricule au ventricule. L'algorithme définit en 16 qu'il y a tachycardie ventriculaire (VT).

En variante, en 15, le pic PR pourrait être divisé par le total de RR. La décision serait alors basée sur la stabilité absolue de l'intervalle PR, plutôt que sur la stabilité de l'intervalle PR par rapport à celle de l'intervalle RR.

Le pic PR est divisé en 17 par le total de PR. Si le rapport est inférieur à un rapport prédéterminé qui peut être programmable et qui est de préférence égal à 75%, alors la conduction de l'oreillette au ventricule est du type "n : 1", et il y a une risque faible de confusion avec une conduction du ventricule vers l'oreillette. L'algorithme définit en 14 qu'il y a tachycardie supraventriculaire (SVT).

En variante, lorsque le nombre d'événements comptés dans la seconde chambre divisé par le nombre d'événements comptés dans la première chambre est supérieur à un rapport prédéterminé, de préférence égal à 1,33, le procédé définit que l'association est en n : 1.

En 18, il y a stabilité de l'intervalle RR et stabilité de l'intervalle PR, avec conduction en 1 : 1. Cette situation pourrait correspondre à une tachyarythmie auriculaire conduite au ventricule, ou à une tachyarythmie ventriculaire conduite à l'oreillette.

Le dispositif selon l'invention peut appliquer un autre critère pour déterminer l'origine de la tachyarythmie. A cet effet, l'accélération du rythme ventriculaire est comparée à une limite prédéterminée, qui peut être programmable et qui est de préférence égale à 25%. Si l'accélération n'a jamais dépassé cette limite, alors il est probable qu'il y a tachycardie sinusale (ST), ce que définit l'algorithme en 19.

Le dispositif selon l'invention examine en 20 s'il y a une dissociation auriculo-ventriculaire au début de l'accélération ventriculaire. Si c'est le cas, alors il est probable que la tachyarythmie a une origine ventriculaire et l'algorithme définit en 16 qu'il y a tachycardie ventriculaire (VT). Dans le cas contraire, il définit en 14 qu'il y a tachycardie supraventriculaire (SVT).

En 21, 22 et 23 sont prévues les commandes de la thérapie appropriée, respectivement au cas de la fibrillation ventriculaire (VF), de la tachycardie ventriculaire (VT) et de la tachycardie supra-ventriculaire (SVT).

Enfin l'algorithme prévoit en 24 l'attente du cycle suivant pour revenir au point 1.

Après la remise à zéro des histogrammes en 6, l'algorithme peut être en attente pendant un nombre prédéterminé de cycles pour la mise à jour des histogrammes. Ce nombre prédéterminé de cycles peut être programmable, et il est de préférence de l'ordre de 8.

Bien que l'invention ait été décrite avec référence à un mode particulier de réalisation, il doit être compris que ce mode de réalisation est seulement illustratif de l'application des principes de l'invention. De nombreuses modifications peuvent être faites sans sortir du cadre de la présente invention, comme defini dans les revendications.

## Revendications

1. Dispositif médical d'analyse de l'activité cardiaque par détection des signaux auriculaires et ventriculaires, correspondant à des événements cardiaques, pour déterminer si une tachyarythmie est susceptible d'être interrompue par stimulation, caractérisé en ce qu'il effectue :
- la détermination du nombre maximal, appelé pic d'autocorrélation, des intervalles récents entre les signaux cardiaques détectés dans une première chambre cardiaque, satisfaisant à des critères prédéterminés de stabilité, et
- la détermination du nombre maximal, appelé pic d'intercorrélation des signaux cardiaques récents détectés dans ladite première chambre cardiaque et correspondant aux mêmes événements que précédemment, qui sont précédés par des signaux cardiaques détectés dans la seconde chambre, dans des limites prédéterminées de temps de conduction.

2. Dispositif selon la revendication 1, caractérisé en ce que la détermination du pic d'autocorrélation comprend les étapes suivantes :
. maintien d'un histogramme d'autocorrélation, c'est-à-dire d'un histogramme des intervalles récents dans ladite première chambre ;
. maintien d'un total d'autocorrélation, c'est-à-dire de la somme du nombre total des comptages dans l'histogramme d'autocorrélation, et
. balayage de l'histogramme d'autocorrélation avec une fenêtre prédéterminée de stabilité pour déterminer le nombre maximal de comptages dans la fenêtre pour toutes les positions possibles de la fenêtre dans l'histogramme.

3. Dispositif selon la revendisation 2, caractérisé en se que la détermination du pic d'intercorrélation comprend les étapes suivantes :
. maintien d'un histogramme d'intercorrélation, c'est-a-dire d'un histogramme des intervalles récents de conduction de ladite seconde chambre vers ladite première chambre, correspondant aux mêmes événements que ceux de l'histogramme d'autocorrélation,
. maintien d'un total d'intercorrélation, c'est-a-dire de la somme du nombre total de comptages dans l'histogramme d'intercorrélation, et
. balayage de l'histogramme d'intercorrélation avec une fenêtre prédéterminée de temps de conduction pour déterminer le nombre maximal de comptages dans la fenêtre pour toutes les positions possibles de la fenêtre dans l'histogramme.

4. Dispositif selon l'une des revendications 2 ou 3, caractérisé en ce que l'étape de maintien dudit histogramme des intervalles récents comprend les étapes suivantes :
. soustraction dans l'histogramme de tout comptage correspondant à un intervalle survenu antérieurement à un nombre prédéterminé de cycles, un cycle correspondant à un signal détecté dans ladite première chambre, et
. addition à l'histogramme d'un comptage correspondant à chaque intervalle survenu pendant le cycle le plus récent.

5. Dispositif selon la revendication 4, caractérisé en ce que le nombre prédéterminé de cycles est programmable.

6. Dispositif selon la revendication 4, caractérisé en ce que le nombre prédéterminé de cycles est d'environ 16.

7. Dispositif selon la revendication 2, caractérisé en ce que dans l'histogramme d'autocorrélation, les intervalles récents dans la première chambre sont triés dans des cases en fonction de leur longueur.

8. Dispositif selon la revendication 3, caractérisé en ce que dans l'histogramme d'intercorrélation, les intervalles récents de conduction de la seconde chambre vers la première chambre sont triés dans des cases en fonction de leur longueur.

9. Dispositif selon l'une des revendications 2 ou 3, caractérisé en ce que ladite fenêtre consiste en un nombre programmable de cases contiguës.

10. Dispositif selon l'une des revendications 2 ou 3, caractérisé en ce que ladite fenêtre consiste en un nombre de cases contiguës correspondant à une variation d'intervalle d'environ 64 ms.

11. Dispositif selon l'une des revendications 7 ou 8, caractérisé en ce que la largeur d'une case dans un histogramme est d'environ 16 ms.

12. Dispositif selon la revendication 1, caractérisé en ce que ladite analyse n'est pas effectuée pour les intervalles dans ladite première chambre qui sont plus longs qu'une limite prédéterminée.

13. Dispositif selon la revendication 12, caractérisé en ce que ladite limite est programmable.

14. Dispositif selon la revendication 12, caractérisé en ce que ladite limite est d'environ 600 ms.

15. Dispositif selon la revendication 12, caractérisé en ce que les histogrammes sont remis à zéro lorsqu'aucun événement n'y a été inscrit pendant un nombre prédéterminé de cycles dans ladite première chambre.

16. Dispositif selon la revendication 15, caractérisé en ce que ledit nombre prédéterminé de cycles est programmable.

17. Dispositif selon la revendication 15, caractérisé en ce que ledit nombre prédéterminé de cycles est d'environ 16.

18. Dispositif selon la revendication 2, caractérisé en ce que l'histogramme d'autocorrélation comprend tous les intervalles entre environ 125 ms et environ 600 ms.

19. Dispositif selon la revendication 3, caractérisé en ce que l'histogramme d'intercorrélation comprend tous les intervalles de conduction entre environ 16 et environ 500 ms.

20. Dispositif selon la revendication 1, caractérisé en ce qu'il effectue un triage des rythmes cardiaques en fonction du pic d'autocorrélation, comprenant les étapes de :
. définir qu'il y a stabilité dans la première chambre lorsque le pic d'autocorrélation, divisé par le total d'autocorrélation excède un rapport prédéterminé de stabilité, et
. définir que la tachyarythmie n'est pas susceptible d'être interrompue par stimulation dans la première chambre lorsqu'il n'y a pas de stabilité dans la première chambre.

21. Dispositif selon la revendication 1, caractérisé en ce qu'il effectue un triage des rythmes cardiaques en fonction du pic d'autocorrélation et du pic d'intercorrélation, comprenant les étapes de :
. définir qu'il y a instabilité de conduction lorsque le pic d'intercorrélation, divisé par le pic d'autocorrélation, n'excède pas un rapport prédéterminé de conduction, et
. définir que la tachyarythmie est susceptible d'être interrompue par stimulation dans la première chambre lorsqu'il y a stabilité dans la première chambre et instabilité de conduction.

22. Dispositif selon la revendication 1, caractérisé en ce qu'il effectue un triage des rythmes cardiaques en fonction des valeurs relatives du pic d'intercorrélation et du total d'autocorrélation, comprenant les étapes de :
. définir qu'il y a instabilité de conduction lorsque le pic d'intercorrélation, divisé par le total d'autocorrélation, n'excède pas un rapport prédéterminé de conduction, et
. définir que la tachyarythmie est susceptible d'être interrompue par stimulation dans la première chambre lorsqu'il y a stabilité dans la première chambre et instabilité de conduction.

23. Dispositif selon l'une des revendications 21 ou 22 caractérisé en ce qu'il trie encore les rythmes cardiaques avec une stabilité définie dans la première chambre et une stabilité de conduction définie, en définissant en outre qu'il y a une conduction en 1 : 1 lorsque le pic d'intercorrélation, divisé par le total d'intercorrélation excède un rapport prédéterminé.

24. Dispositif selon l'une des revendications 21 ou 22, caractérisé en ce qu'il trie encore les rythmes cardiaques avec une stabilité, définie dans la première chambre, en définissant en outre qu'il y a une conduction en n : 1 lorsque le total des événements dans la deuxième chambre, divisé par le total d'autocorrélation excède un rapport prédéterminé.

25. Dispositif selon l'une des revendications 20 à 24, caractérisé en ce que lesdits rapports prédéterminés sont programmables.

26. Dispositif selon l'une des revendications 20 à 23, caractérisé en ce que lesdits rapports prédéterminés sont d'environ 75%.

27. Dispositif selon la revendication 24, caractérisé en ce que ledit rapport prédéterminé est d'environ 1,33.

28. Dispositif selon l'une des revendications 20 à 23, caractérisé en ce qu'il trie les rythmes cardiaques seulement lorsque la longueur des intervalles dans ladite première chambre tombe entre une limite inférieure prédéterminée et une limite supérieure prédéterminée.

29. Dispositif selon la revendication 28, caractérisé en ce que lesdites limites des intervalles sont programmables.

30. Dispositif selon la revendication 28, caractérisé en ce que la limite inférieure prédéterminée est d'environ 375 ms et la limite supérieure prédéterminée est d'environ 600 ms.

31. Dispositif selon l'une des revendications 20 à 24, caractérisé en ce qu'il attend pour trier les rythmes jusqu'à ce que les histogrammes aient été mis à jour pour un nombre minimal prédéterminé de cycles.

32. Dispositif selon la revendication 31, caractérisé en ce que ledit nombre prédéterminé de cycles est programmable.

33. Dispositif selon la revendication 31, caractérisé en ce que ledit nombre prédéterminé de cycles est d'environ 8.

34. Dispositif selon la revendication 31, caractérisé en ce qu'il effectue un triage encore des rythmes cardiaques avec stabilité définie dans la première chambre et stabilité de conduction définie, comprenant les étapes de:
. identifier l'occurrence d'une accélération de la fréquence dans la première chambre, excédant une limite d'accélération prédéterminée ;
. identifier l'occurrence d'une dissociation de conduction entre les deux chambres lors de la détection de ladite accélération, et
. définir que la tachyarythmie est susceptible d'être interrompue par stimulation dans la première chambre lorsqu'il y a accélération et dissociation.

35. Dispositif selon la revendication 34, caractérisé en ce qu'il définit que la tachyarythmie est susceptible d'être interrompue par stimulation seulement lorsqu'elle est définie en 1 : 1.

36. Dispositif selon l'une des revendications 21 à 35, caractérisé en ce qu'il délivre une stimulation, lorsque la tachyarythmie a été définie comme susceptible d'être interrompue par stimulation.

## Claims

1. A medical device for analysing a cardiac activity by detecting auricular and ventricular signals corresponding to cardiac events in order to determine whether a tachyarhythmia might be terminable by stimulation, characterised in that it provides for:
- the determination of the maximal number, called auto-correlation peak, of the recent intervals between the cardiac signals detected in a first cardiac chamber and which meet determining stability criteria, and
- the determination of the maximal number, called cross-correlation peak, of the recent cardiac signals detected in said first cardiac chamber and which correspond to the same events as those previously mentioned, which are preceded by cardiac signals detected in the second chamber, within determining conduction time limits.

2. The device as claimed in claim 1, characterised in that the determination of the auto-correlation peak comprises the steps of:
- maintaining an histogram, i. e. a histogram of the recent intervals in said first chamber;
- maintaining a auto-correlation total, i. e. the sum of total number of counts in the auto-correlation histogram, and
- sweeping the auto-correlation histogram with a predetermined window of stability to determine the maximum number of counts in the window for all possible positions of the window in the histogram.

3. The device as claimed in claim 2, characterised in that the determination of the cross-correlation peak comprises the steps of:
- maintaining a cross-correlation histogram, i. e. a histogram of recent intervals of conduction from said second chamber to said first chamber, corresponding to the same events as those in the auto-correlation histogram,
- maintaining a cross-correlation total, i. e. the sum of the total number of counts in the cross-correlation histogram, and
- sweeping the cross-correlation histogram with a predetermined window of conduction time to determine the maximum number of counts in the window for all possible positions of the window in the histogram.

4. The device as claimed in claim 2 or 3, characterised in that the step of maintaining said histogram of recent intervals comprises the following steps:
- subtraction from the histogram of any count corresponding to an interval occurring before a predetermined number of cycles, wherein a cycle corresponds to a signal detected in said first chamber, and
- addition to the histogram of a count corresponding to each interval having occurred during the most recent cycle.

5. The device as claimed in claim 4, characterised in that the number of predetermined cycles is programmable.

6. The device as claimed in claim 4, characterised in that the number of predetermined cycles is approximately 16.

7. The device as claimed in claim 2, characterised in that in the auto-correlation histogram, the recent intervals in the first chamber are sorted in bins according to their lengths.

8. The device as claimed in claim 3, characterised in that in the cross-correlation histogram, the recent intervals of conduction from the second chamber to the first chamber are sorted in bins according to their lengths.

9. The device as claimed in claim 2 or 3, characterised in that said window consists in a programmable number of contiguous bins.

10. The device as claimed in claim 2 or 3, characterised in that said window consists in a number of contiguous bins corresponding to an interval variation of approximately 64 ms.

11. The device as claimed in claim 7 or 8, characterised in that the width of a bin in a histogram is approximately 16 ms.

12. The device as claimed in claim 1, characterised in that said analysis is not undertaken for intervals of the first chamber that are longer than a predetermined limit.

13. The device as claimed in claim 12, characterised in that said limit is programmable.

14. The device as claimed in claim 12, characterised in that said limit is approximately 600 ms.

15. The device as claimed in claim 12, characterised in that the histograms are reset to zero if no event has been registered during a predetermined number of cycles in said first chamber.

16. The device as claimed in claim 15, characterised in that said number of predetermined cycles is programmable.

17. The device as claimed in claim 15, characterised in that said number of predetermined cycles is approximately 16.

18. The device as claimed in claim 2, characterised in that the auto-correlating histogram comprises all intervals between approximately 125 ms and approximately 600 ms.

19. The device as claimed in claim 3, characterised in that the cross-correlating histogram comprises all conduction intervals between approximately 16 ms and approximately 500 ms.

20. The device as claimed in claim 1, characterised in that it provides for sorting the cardiac rhythms as a function of the auto-correlation peak, comprising the steps of:
- determining that there is stability in the first chamber when the auto-correlation peak, divided by the total auto-correction exceeds a predetermined stability quotient, and
- determining that the tachyarhythmia is not susceptible to be terminated by a stimulation in the first chamber in the absence of stability in the first chamber.

21. The device as claimed in claim 1, characterised in that provides for sorting the cardiac rhythms as a function of the auto-correlation peak and the cross-correlation peak, comprising the steps of:
- defining a conduction instability when the cross-correlation peak divided by the auto-correlation peak does not exceed a predetermined conduction quotient, and
- determining that the tachyarhythmia is susceptible to be terminated by stimulation in the first chamber when there is stability in the first chamber and conduction instability.

22. The device as claimed in claim 1, characterised in that provides for sorting the cardiac rhythms as a function of the relative values of the auto-correlation total and the cross-correlation peak, comprising the steps of:
- determining a conduction instability when the cross-correlation peak divided by the auto-correlation total does not exceed a predetermined conduction quotient; and
- determining that the tachyarhythmia is susceptible to be terminated by stimulation in the first chamber when there is stability in the first chamber and conduction instability.

23. The device as claimed in claim 21 or 22, characterised in that it further provides for sorting the cardiac rhythms with a stability determined in the first chamber and a determined conduction stability, by furthermore determining if there is a 1:1 ratio when the cross-correlation peak divided by the total of cross-correlation exceeds a predetermined quotient.

24. The device as claimed in claim 21 or 22, characterised in that it further provides for sorting the cardiac rhythms with a stability determined in the first chamber, by furthermore determining if there is conduction with a n:1 ratio when the total count of events in the second chamber divided by the auto-correlation total exceeds a predetermined quotient.

25. The device as claimed in any one of claims 20-24, characterised in that said predetermined quotients are programmable.

26. The device as claimed in any one of claims 20-23, characterised in that said predetermined quotients are approximately 75%.

27. The device as claimed in claim 24, characterised in that said predetermined quotient is approximately 1.33.

28. The device as claimed in any one of claims 20-23, characterised in that it sorts the cardiac rhythms only when the intervals in said first chamber are within a minimum and a maximum predetermined limits.

29. The device as claimed in claim 28, characterised in that said limits of the intervals are programmable.

30. The device as claimed in claim 28, characterised in that the predetermined minimum limit is approximately 375 ms and the predetermined maximum limit is approximately 600 ms.

31. The device as claimed in any one of claims 20-24, characterised in that it suspends sorting the rhythms until the histograms have been updated with regard to a predetermined minimum number of cycles.

32. The device as claimed in claim 31, characterised in that the predetermined number of cycles is programmable.

33. The device as claimed in claim 31, characterised in that the predetermined number of cycles is approximately 8.

34. The device as claimed in claim 31, characterised in that it provides for further sorting the cardiac rhythms when there is determined stability in the first chamber and determined conduction stability, comprising the steps of:
- identifying the occurrence of an acceleration of the frequency in the first chamber, which is greater than a predetermined acceleration limit;
- identifying the occurrence of a dissociation of conduction between the two chambers during the detection of said acceleration, and
- determining that the tachyarhythmia is susceptible to be terminated by a stimulation in the first chamber when there is both acceleration and dissociation.

35. The device as claimed in claim 34, characterised in that it determines that the tachyarhythmia is susceptible to be terminated by stimulation only when it is determined by a 1:1 ratio.

36. The device as claimed in any one of claims 21-35, characterised in that gives a stimulation when the tachyarhythmia has been defined as susceptible to be terminated by stimulation.

## Patentansprüche

1. Medizinische Vorrichtung zur Analyse der Herzaktivität mittels Erfassung von Herzereignissen entsprechenden aurikulären und ventrikulären Signalen, zur Bestimmung, ob eine Tachyarrythmie durch Stimulation unterbrochen werden kann, **dadurch gekennzeichnet, daß sie folgendes bewirkt**
- die Bestimmung der als Autokorrelationspeak bezeichneten maximalen Anzahl neuester Intervalle zwischen den in einer ersten Herzkammer erfaßten Herzsignalen, die vorbestimmten Stabilitätskriterien genügen, und
- die Bestimmung der als Interkorrelationspeak bezeichneten maximalen Anzahl neuester Herzsignale, die in der ersten Herzkammer erfaßt wurden und die den selben Ereignissen wie zuvor entsprechen, denen innerhalb der vorbestimmten Grenzwerte der Leitungszeit in der zweiten Kammer erfaßte Herzsignale vorausgehen.

2. Vorrichtung nach Anspruch 1, **dadurch gekennzeichnet, daß** die Bestimmung des Autokorrelationspeak die folgenden Schritte umfaßt:
- Führen eines Autokorrelations-Histogramms, also eines Histogramms der neuesten Intervalle in der ersten Kammer,
- Führen einer Gesamt-Autokorrelation, also der Summe der Gesamtanzahl der Zählungen in dem Autokorrelations-Histogramm, und
- Abfragen des Autokorrelations-Histogramms mit einem vorbestimmten Stabilitätsfenster zur Bestimmung der maximalen Anzahl von Zählungen in dem Fenster für alle möglichen Positionen des Fensters in dem Histogramm.

3. Vorrichtung nach Anspruch 2, **dadurch gekennzeichnet, daß** die Bestimmung des Interkorrelationspeak die folgenden Schritte umfaßt:
- Führen eines Interkorrelations-Histogramms, also eines Histogramms der neuesten Intervalle der Leitung von der zweiten Kammer zu der ersten Kammer, die den selben Ereignissen wie jenen des Autokorrelations-Histogramms entsprechen,
- Führen einer Gesamt-Interkorrelation, also der Summe der Gesamtanzahl der Zählungen in dem Interkorrelations-Histogramm, und
- Abfragen des Interkorrelations-Histogramms mit einem vorbestimmten Leitungszeitfenster zur Bestimmung der maximalen Anzahl von Zählungen in dem Fenster für alle möglichen Positionen des Fensters in dem Histogramm.

4. Vorrichtung nach einem der Ansprüche 2 oder 3, **dadurch gekennzeichnet, daß** der Schritt des Führens des Histogramms der neuesten Intervalle die folgenden Schritte umfaßt:
- Subtraktion jeder Zählung vom Histogramm, die einem Intervall entspricht, das vor einer vorbestimmten Anzahl von Zyklen aufgetreten ist, wobei ein Zyklus einem in der ersten Kammer erfaßten Signal entspricht, und
- Addition einer Zählung zum Histogramm, die jedem Intervall entspricht, das während des allerneuesten Zyklus aufgetreten ist.

5. Vorrichtung nach Anspruch 4, **dadurch gekennzeichnet, daß** die vorbestimmte Anzahl von Zyklen programmierbar ist.

6. Vorrichtung nach Anspruch 4, **dadurch gekennzeichnet, daß** die vorbestimmte Anzahl von Zyklen etwa 16 beträgt.

7. Vorrichtung nach Anspruch 2, **dadurch gekennzeichnet, daß** im Autokorrelations-Histogramm die neuesten Intervalle in der ersten Kammer abhängig von ihrer Länge in Felder sortiert werden.

8. Vorrichtung nach Anspruch 3, **dadurch gekennzeichnet, daß** im Interkorrelations-Histogramm die neuesten Intervalle der Leitung von der zweiten Kammer zu der ersten Kammer abhängig von ihrer Länge in Felder sortiert werden.

9. Vorrichtung nach einem der Ansprüche 2 oder 3, **dadurch** g**ekennzeichnet, daß** das Fenster aus einer programmierbaren Anzahl von benachbarten Feldern besteht.

10. Vorrichtung nach einem der Ansprüche 2 oder 3, **dadurch gekennzeichnet, daß** das Fenster aus einer Anzahl von benachbarten Feldern besteht, die einer Änderung des Intervalls von etwa 64 ms entspricht.

11. Vorrichtung nach einem der Ansprüche 7 oder 8, **dadurch gekennzeichnet, daß** die Größe eines Feldes in einem Histogramm etwa 16 ms beträgt.

12. Vorrichtung nach Anspruch 1, **dadurch gekennzeichnet, daß** die Analyse nicht für die Intervalle in der ersten Kammer erfolgt, die länger als ein vorbestimmter Grenzwert sind.

13. Vorrichtung nach Anspruch 12, **dadurch gekennzeichnet, daß** der Grenzwert programmierbar ist.

14. Vorrichtung nach Anspruch 12, **dadurch gekennzeichnet, daß** der Grenzwert etwa 600 ms beträgt.

15. Vorrichtung nach Anspruch 12, **dadurch gekennzeichnet, daß** die Histogramme auf Null zurückgesetzt werden, wenn während einer vorbestimmten Anzahl von Zyklen in der ersten Kammer kein Ereignis in die Histogramme eingetragen wurde.

16. Vorrichtung nach Anspruch 15, **dadurch gekennzeichnet, daß** die vorbestimmte Anzahl von Zyklen programmierbar ist.

17. Vorrichtung nach Anspruch 15, **dadurch gekennzeichnet, daß** die vorbestimmte Anzahl von Zyklen etwa 16 beträgt.

18. Vorrichtung nach Anspruch 2, **dadurch gekennzeichnet, daß** das Autokorrelations-Histogramm alle Intervalle zwischen etwa 125 ms und etwa 600 ms umfaßt.

19. Vorrichtung nach Anspruch 3, **dadurch gekennzeichnet, daß** das Interkorrelations-Histogramm alle Leitungsintervalle zwischen etwa 16 und etwa 500 ms umfaßt.

20. Vorrichtung nach Anspruch 1, **dadurch gekennzeichnet, daß** sie ein Sortieren der Herzrhythmen in Abhängigkeit des Autokorrelationspeak durchführt, das die folgenden Schritte umfaßt:
- Definieren, daß Stabilität in der ersten Kammer vorliegt, wenn der Autokorrelationspeak dividiert durch die Gesamt-Autokorrelation ein vorbestimmtes Stabilitätsverhältnis überschreitet, und
- Definieren, daß die Tachyarrythmie nicht durch Stimulation in der ersten Kammer unterbrochen werden kann, wenn in der ersten Kammer keine Stabilität vorliegt.

21. Vorrichtung nach Anspruch 1, **dadurch gekennzeichnet, daß** sie ein Sortieren der Herzrhythmen in Abhängigkeit des Autokorrelationspeak und des Interkorrelationspeak durchführt, das die folgenden Schritte umfaßt:
- Definieren, daß Instabilität der Leitung vorliegt, wenn der Interkorrelationspeak dividiert durch den Autokorrelationspeak ein vorbestimmtes Leitungsverhältnis nicht überschreitet, und
- Definieren, daß die Tachyarrythmie durch Stimulation in der ersten Kammer unterbrochen werden kann, wenn Stabilität in der ersten Kammer und Instabilität der Leitung vorliegt.

22. Vorrichtung nach Anspruch 1, **dadurch gekennzeichnet, daß** sie ein Sortieren der Herzrhythmen in Abhängigkeit der Relativwerte des Interkorrelationspeak und der Gesamt-Autokorrelation durchführt, das die folgenden Schritte umfaßt:
- Definieren, daß Instabilität der Leitung vorliegt, wenn der Interkorrelationspeak dividiert durch die Gesamt-Autokorrelation ein vorbestimmtes Leitungsverhältnis nicht übersteigt, und
- Definieren, daß die Tachyarrythmie durch Stimulation in der ersten Kammer unterbrochen werden kann, wenn Stabilität in der ersten Kammer und Instabilität der Leitung vorliegt.

23. Vorrichtung nach einem der Ansprüche 21 oder 22, **dadurch gekennzeichnet, daß** sie zudem die Herzrhythmen mit einer definierten Stabilität in der ersten Kammer und einer definierten Stabilität der Leitung sortiert, wobei unter anderem definiert wird, daß eine Leitung von 1:1 vorliegt, wenn der Interkorrelationspeak dividiert durch die Gesamt-Interkorrelation ein vorbestimmtes Verhältnis überschreitet.

24. Vorrichtung nach einem der Ansprüche 21 oder 22, **dadurch gekennzeichnet, daß** sie zudem die Herzrhythmen mit einer definierten Stabilität in der ersten Kammer sortiert, wobei unter anderem definiert wird, daß eine Leitung von n:1 vorliegt, wenn die Gesamtheit der Ereignisse in der zweiten Kammer dividiert durch die Gesamt-Autokorrelation ein vorbestimmtes Verhältnis überschreitet.

25. Vorrichtung nach einem der Ansprüche 20 bis 24, **dadurch gekennzeichnet, daß** die vorbestimmten Verhältnisse programmierbar sind.

26. Vorrichtung nach einem der Ansprüche 20 bis 23, **dadurch gekennzeichnet, daß** die vorbestimmten Verhältnisse etwa 75% betragen.

27. Vorrichtung nach Anspruch 24, **dadurch gekennzeichnet, daß** das vorbestimmte Verhältnis etwa 1,33 beträgt.

28. Vorrichtung nach einem der Ansprüche 20 bis 23, **dadurch gekennzeichnet, daß** sie die Herzrhythmen lediglich dann sortiert, wenn die Länge der Intervalle in der ersten Kammer zwischen einen vorbestimmten unteren Grenzwert und einen vorbestimmten oberen Grenzwert fällt.

29. Vorrichtung nach Anspruch 28, **dadurch gekennzeichnet, daß** die Grenzwerte der Intervalle programmierbar sind.

30. Vorrichtung nach Anspruch 28, **dadurch gekennzeichnet, daß** der untere vorbestimmte Grenzwert etwa 375 ms und der obere vorbestimmte Grenzwert etwa 600 ms beträgt.

31. Vorrichtung nach einem der Ansprüche 20 bis 24, **dadurch gekennzeichnet, daß** sie mit dem Sortieren der Rhythmen wartet, bis die Histogramme für eine vorbestimmte minimale Anzahl von Zyklen aktualisiert worden sind.

32. Vorrichtung nach Anspruch 31, **dadurch gekennzeichnet, daß** die vorbestimmte Anzahl von Zyklen programmierbar ist.

33. Vorrichtung nach Anspruch 31, **dadurch gekennzeichnet, daß** die vorbestimmte Anzahl von Zyklen etwa 8 beträgt.

34. Vorrichtung nach Anspruch 31, **dadurch gekennzeichnet, daß** sie zudem ein Sortieren der Herzrhythmen mit definierter Stabilität in der ersten Kammer und definierter Stabilität der Leitung vornimmt, das die folgenden Schritte umfaßt:
- Identifizieren des Auftretens einer Beschleunigung der Frequenz in der ersten Kammer, die einen vorbestimmten Grenzwert der Beschleunigung überschreitet,
- Identifizieren des Auftretens einer Dissoziation der Leitung zwischen den zwei Kammern bei der Erfassung der Beschleunigung, und
- Definieren, daß die Tachyarrhythmie durch Stimulation in der ersten Kammer unterbrochen werden kann, wenn eine Beschleunigung und eine Dissoziation vorliegt.

35. Vorrichtung nach Anspruch 34, **dadurch gekennzeichnet, daß** sie definiert, daß die Tachyarrhythmie nur durch Stimulation unterbrochen werden kann, wenn sie als 1:1 definiert ist.

36. Vorrichtung nach einem der Ansprüche 21 bis 35, **dadurch gekennzeichnet, daß** sie eine Stimulation abgibt, wenn die Tachyarrhythmie als durch Stimulation unterbrechbar definiert ist.
